(19) Europäisches Patentamt
European Patent Office
Office européen des brevets

(11) Publication number: **0 052 459**
**B1**

(12) **EUROPEAN PATENT SPECIFICATION**

(45) Date of publication of patent specification: **04.12.85**

(51) Int. Cl.[4]: **C 08 G 63/06**, C 12 P 7/62 // C08L27/06

(21) Application number: **81305186.9**

(22) Date of filing: **30.10.81**

(54) **Beta-hydroxybutyrate polymers.**

(30) Priority: **18.11.80 GB 8036967**
**07.07.81 GB 8120991**

(43) Date of publication of application:
**26.05.82 Bulletin 82/21**

(45) Publication of the grant of the patent:
**04.12.85 Bulletin 85/49**

(84) Designated Contracting States:
**BE CH DE FR GB IT LI NL SE**

(56) References cited:
**GB-A-1 207 588**
**US-A-3 275 610**

**ENVIRONMENTAL SCIENCE & TECHNOLOGY Vol. 8, No. 6, June 1974 Washington WALLEN et al. "Poly-Beta-hydroxyalkanoate from Activated Sludge" pages 576 to 579**

**The file contains technical information submitted after the application was filed and not included in this specification**

(73) Proprietor: **IMPERIAL CHEMICAL INDUSTRIES PLC**
**Imperial Chemical House Millbank**
**London SW1P 3JF (GB)**

(72) Inventor: **Holmes, Paul Arthur**
**39 Skottowe Crescent**
**Great Ayton Middlesbrough Cleveland (GB)**
Inventor: **Wright, Leonard Frederick**
**8 Blairmore Gardens**
**Eaglescliffe Cleveland (GB)**
Inventor: **Collins, Stephen Hugh**
**Belcroft Cottage**
**Cowesby Thirsk North Yorkshire (GB)**

(74) Representative: **Gratwick, Christopher et al**
**Imperial Chemical Industries PLC Legal Department: Patents PO Box 6 Bessemer Road**
**Welwyn Garden City Herts, AL7 1HD (GB)**

**EP 0 052 459 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to β-hydroxybutyrate (HB) polymers: poly(β-hydroxybutyric acid) is hereinafter referred to as PHB.

PHB is accumulated by various micro-organisms, principally bacteria, as an energy reserve material as granules within the microbial cells.

PHB extracted from such cells is a thermoplastic polyester of the repeat structure

$$—O . CH(CH_3) . CH_2 . CO—$$

that rapidly crystallises to a relatively high level e.g. of the order of 70% or more. This crystallisation behaviour is often disadvantageous when the polymer is to be used as, for example, a moulding material.

We have found that the crystallisation of PHB can be modified by incorporation of units of a dissimilar monomer into the polymer chain.

In the following description of the metabolic pathways leading to the polymer synthesis, the following abbreviations are employed:

CoASH is unesterified Coenzyme A. So $CH_3 . CO . S . CoA$ is the acetyl thioester of Coenzyme A and is more commonly termed acetyl CoA.

NADP is nicotinamide adenine dinucleotide in the oxidised state. $NADPH_2$ is reduced NADP.

It is believed that, in the biosynthesis of PHB by a micro-organism the first step is the synthesis of acetyl CoA. This can be formed, for example, from Coenzyme A and acetate, or by the decarboxylation of pyruvate, which is a product of the glycolysis of carbohydrates, or which can be formed by decarboxylation of oxaloacetate, the latter being a member of the tricarboxylic acid, TCA, cycle, otherwise known as the Krebs cycle.

Thus with acetate as the source of acetyl CoA, the PHB is produced by a metabolic pathway involving the reactions:

1. $CH_3 . CO . O^- + CoA . SH \xrightarrow{\text{thiokinase}} CH_3 . CO . S . CoA + OH^-$

2. $2CH_3 . CO . S . CoA \xrightarrow{\beta\text{-ketothiolase}} CH_3 . CO . CH_2 . CO . S . CoA + CoA . SH$

3. $CH_3 . CO . CH_2 . CO . S . CoA + NADPH_2 \xrightarrow{\text{reductase}} CH_3 . CHOH . CH_2 . CO . S . CoA + NADP$
   β- hydroxybutyryl CoA

4. $CH_3 . CHOH . CH_2 . CO . S . CoA + (—O . CH(CH_3) . CH_2 . CO—)_{n-1} \xrightarrow{\text{polymerase}} (—O . CH(CH_3) . CH_2 . CO—)_n + CoA . SH$

where $(—O . CH(CH_3) . CH_2 . CO—)_{n-1}$ is a PHB molecule containing n-1 repeat units. Thus reaction 4 adds a $—O . CH(CH_3) . CH_2 . CO—$ unit to the polymer chain.

We have found that a minor proportion of comonomer units may be introduced into the polymer chain by cultivation of the micro-organism under certain conditions in the presence of certain organic acids. to be of any practical use as plastics materials, the polymers should have a weight average molecular weight, (Mw), above 10,000 e.g. as measured by gel permeation chromatography.

Accordingly, we provide HB copolymers containing 50 to 99 mole % of β-hydroxybutyrate repeat units

$$—O . CH(CH_3) . CH_2 . CO—$$

and 1 to 50 mole % of β-hydroxyvalerate (HV) repeat units

$$—O . CH(C_2H_5) . CH_2 . CO—$$

said copolymers having (i) a weight average molecular weight above 10,000 and the D(–) configuration or (ii) a weight average molecular weight above 50,000.

Such copolymers may be produced since the enzymes involved have a degree of non-specificity.

Thus the enzyme, thiokinase, involved in reaction 1 has a broad specificity: it will, attach Coenzyme A to a variety of other carboxylate groups according to the general reaction

1a. $R . CO . O^- + CoA . SH \xrightarrow{\text{thiokinase}} R . CO . S . CoA + OH^-$

e.g. $CH_3 . CH_2 . CO . O^- + CoA . SH \rightarrow CH_3 . CH_2 . CO . S . CoA + OH^-$ propionyl CoA

Reaction 2, involving the enzyme β-ketothiolase, can be rewritten

2. $CH_3 . CO . S . CoA + CH_3 . CO . S . CoA \rightarrow CH_3 . CO . CH_2 . CO . S . CoA + CoA . SH$.

This reaction appears to be partially specific in that one reactant must be acetyl CoA. The general reaction is thus

2a. $R . CO . S . CoA + CH_3 . CO . S . CoA \rightarrow R . CO . CH_2 . CO . S . CoA + CoA . SH$

Likewise the specificity of the reductase enzyme involved in reaction 3 is degenerate and will reduce certain fatty acyl thioesters of the general formula $R . CO . CH_2 . CO . S . CoA$ as follows:

3a. $R . CO . CH_2 . CO . S . CoA + NADPH_2 \rightarrow R . CHOH . CH_2 . CO . S . CoA + NADP$

The polymerase enzyme of reaction 4 is not absolutely specific. The general reaction can be written:

4a. $R . CHOH . CH_2 . CO . S . CoA + (—O . CHR . CH_2 . CO)_n \rightarrow (—O . CHR . CH_2 . CO—)_n—O . CHR . CH_2CO— + CoA . SH$

in which the radicals R may differ.

This route therefore gives rise to polymers containing units of the formula

$$—O . CHR . CH_2 . CO—$$

Hence HB/HV copolymers can be produced if, in some of the repeat units, R is methyl and in others is ethyl.

The β-hydroxyl thioester, e.g. $R . CHOH . CH_2 . CO . S . CoA$, reactant of reaction 4a can also be made, in certain cases, by the reactions catalysed by the non-specific fatty acid metabolism enzyme enoylhydratase:

5a. $R . CH{=}CH . CO . O^- + H_2O \xrightarrow{\text{enoylhydratase}} R . CH(OH) . CH_2CO . O^-$

5b. $R . CH(OH) . CH_2 . CO . O^- + CoASH \rightarrow R . CH(OH) . CH_2 . CO . S . CoA + OH^-$

(The reactions 5a, 5b may in fact be reversed, i.e. the hydration of the carbon-carbon double bond may occur after thioesterification).

Hence units of the formula

$$—O . CHR . CH_2 . CO—$$

can also be introduced into the polymer chain by the use of reactions 5a, 5b and 4a. Hence the copolymers may be produced if in some of the repeat units R is methyl and is ethyl in others.

The proportion of HV repeat units in the copolymer is between 1 and 50, particularly 1 to 40, mole percent of the total repeat units in the copolymer. In some cases the polymer produced by the micro-organism may be a blend of a HB homopolymer with an HB/HV copolymer. In this case the overall proportion of HV repeat units in the polymer should be between 1 and 50 mole percent of the total repeat units. Preferably the proportion of HV repeat units in the polymer is between 3 and 30 mole %.

We have found that, instead of following the course of the above reactions, in some cases the micro-organism will perform elimination reactions in addition to, or instead of, some of the reactions outlined above, giving polymers containing HV units instead of the units that might be expected from the above reaction sequences.

Certain polymers containing HB units and other units have been described in the literature.

Thus Wallen et al describe in "Environmental Science and Technology" *6* (1972) pages 161—164 and *8* (1974) pages 576—579 a polymer melting at 97—100°C (after repeated washing) isolated from activated sludges and containing HB units and HV units in the ratio of 1:5. The polymer thus contains only about 17% of HB units. Marchessault et al report in "IUPAC Macro Florence 1980 International Symposium on Macromoles Preprints" *2* (1980) pages 272—275 a study of this polymer and confirmed that it contained mainly HV units.

United States Patent Specification 3275610 describes the microbiological production of polyesters by cultivating certain micro-organisms, especially *Nocardia salmonicolor*, on carboxylic acids containing 4 carbon atoms. In Examples 2 and 3, where the acids were 3-butenoic and α-hydroxybutyric acids respectively, the polymers appear, from the quoted melting points of the order of 178—184°C, to be PHB. In Example 1 however, wherein 2-methyl acrylic acid, i.e. methacrylic acid, was employed the polymer produced is unidentified but is described has having a melting point of 215—220°C and as being soluble in methyl ethyl ketone. In contrast thereto, HB/HV copolymers in accordance with the present invention, have melting points below 180°C and are insoluble in cold methyl ethyl ketone.

When PHB-accumulating micro-organisms are aerobically cultured on a suitable substrate, i.e. a source of energy and carbon, they reproduce until one or more of the essential requirements for reproduction is exhausted. This reproduction of the micro-organism is hereinafter referred to as growth. Upon exhaustion of an essential growth requirement, further growth occurs only to a very limited extent, if at all, but, providing the substrate is not exhausted, PHB may be accumulated by the micro-organism.

With some micro-organisms, even in the absence of a PHB-inducing constraint such as a limitation on one or more of the essential growth requirements, PHB may also be accumulated while growth of the micro-organism is taking place: however the amount of PHB so accumulated is generally small and typically is less than about 10% by weight of the cells produced. Thus when grown in batch culture, the micro-organism will grow, with little or no PHB accumulation until one or more of the essential requirements for growth becomes exhausted, and then the micro-organism synthesises PHB.

We have found that in order to produce the copolymers it is generally necessary to use the acid that is to give rise to the HV units as at least part of the substrate during cultivation of the micro-organism under conditions wherein the amount of one or more of the essential requirements for growth, but not PHB accumulation, is limited. Where there is no such restriction of an essential requirement for growth, the acid will generally be metabolised by the micro-organism by other pathways leading to e.g. acetyl CoA or to a member of the TCA cycle, and so copolymers will not be produced. Thus, as an example, propionic acid can be metabolised by micro-organisms, in the absence of any growth limitation, via propionyl CoA, with the incorporation of carbon dioxide to methyl malonyl CoA, and thence to succinate, a member of the TCA cycle.

The copolymers may thus be produced by cultivating a micro-organism, that is capable of accumulating the copolymer, in an aqueous medium on a water soluble assimilable carbon containing substrate with at least part of the cultivation being conducted under conditions of limitation of one or more of the essential requirements for microbial growth, but not polymer accumulation, using, during at least part of the period when the cultivation is so limited, a substrate comprising an organic acid, or salt thereof, that is metabolisable by said micro-organism, under said limitation conditions, to said copolymer.

In this regard it is noted that, in United States Patent 3275610 mentioned above, the amounts of cells produced were such that no growth limitation were imposed.

In addition to the substrate and oxygen (which is generally supplied by injecting air into the aqueous medium in the fermenter), various nutrient salts are required to enable the micro-organism to grow. Thus sources of the following elements in assimilable form, normally as water soluble salts, are generally required: nitrogen, phosphorus, sulphur, potassium, sodium, magnesium, calcium, and iron, together with traces of elements such as manganese, zinc and copper. While it may be possible to induce polymer accumulation by restricting the supply of oxygen to the fermenter, it is preferred to restrict the amount of one or more of the nutrient salts. The most practical elements to limit are nitrogen, phosphorus, or, less preferably, magnesium, sulphur or potassium. Of these it is most preferred to restrict the amount of nitrogen (which is conveniently supplied as an ammonium salt). The amount of assimilable nitrogen required is about 8—15% by weight of the desired weight of cells less accumulated polymer.

The fermentation is preferably conducted so that the dry weight of the HB polymer-containing cells is at least 5 g per litre of aqueous medium. Hence if, for example, it is desired to produce 10 g per litre of HB polymer-containing cells having a HB polymer content of 40% by weight, the amount of the essential nutrient fed to the fermenter that is used to limit the amount of cell growth must be that required to support the growth of 6 g per litre of cells containing no HB polymer: thus, if nitrogen is employed as the growth limiting growth limiting nutrient, since the nitrogen content of HB polymer-free bacterial cells is about 8—15% by weight, the amount of assimilable nitrogen required would be between about 0.5 and 0.9 g per litre, e.g. about 0.6 to 1.2 g of ammonium ions per litre.

The fermentation may be conducted under the conditions e.g. pH, temperature, and degree of aeration (unless oxygen is utilised as the limiting nutrient) conventionally used for the micro-organism. Likewise the amounts of nutrient salts (other than the growth limiting nutrient whose amount may be determined following the considerations outlined hereinbefore) employed may be those normally used for growth of the micro-organism.

The micro-organism is preferably grown to a certain desired weight by cultivation in the presence of sufficient of the nutrient required for growth that is to be restricted in the polymer accumulation stage on a readily metabolisable substrate, such as a carbohydrate, and then cultivated under conditions of growth requirement restriction to cause the polymer accumulation. In some cases the substrate for at least part, and in some cases all, of the growth stage may be the acid that gives rise to the HV repeat units in the polymer accumulation stage.

The fermentation may be performed as a batch fermentation in which case polymer accumulation will occur as the amount of the nutrient that is required for growth but not polymer accumulation becomes depleted, i.e. exhausted. Alternatively the fermentation may be conducted as a continuous process wherein aqueous medium containing the bacterial cells is removed, continuously or intermittently, from the fermentation vessel at a rate corresponding to the rate of addition of fresh aqueous medium and substrate thereto. It is preferred that the amount of the nutrient that is restricted that is fed to the fermentation vessel is such that the aqueous medium removed from the vessel contains little or none of that nutrient, and the aqueous medium removed from the vessel is then fed to a second fermentation vessel, operated either in

batch or, preferably, continuous fashion wherein polymer accumulation is caused to take place by continuing the aerobic cultivation with the addition of a fresh quantity of substrate comprising the copolymer producing acid. While additional quantities of substrate and nutrient salts may be added in this further fermentation step, since further growth is generally not desired, little or no further quantity of the nutrient utilised to limit growth should be added. It will however be appreciated that the aqueous medium fed to the further fermenter or fermenters from the first fermenter may contain some residual quantity of the limiting nutrient and/or the addition of a further small quantity thereof may be desirable for efficient operation.

In either a batch process, or a continuous process as described above, the acid used to provide the HV repeat units is used as part, or all, of the substrate during the polymer accumulation stage occurring upon exhaustion of the nutrient required for growth. The acid may be used in admixture with a substrate, e.g. a carbohydrate, that will give HB repeat units, or may be the sole substrate: in the latter case sufficient of the acid will normally be metabolised by other pathways to acetyl CoA to provide the HB repeat units and any acetyl CoA required to produce the HV repeat units, i.e. if a pathway involving reaction 2a, is employed. However, when the acid is the sole substrate, the yield of polymer is often low.

The acid giving HV repeat units may be present for only part of the polymer accumulation stage: for the rest of the polymer accumulation stage, which may occur before and/or after the part of the polymer accumulation stage wherein the acid is present, a substrate giving only HB repeat units may be the sole substrate.

In some cases it may be possible to prevent the "normal" metabolisation of the acid, i.e. to acetyl CoA, by blocking enzymes required for that pathway and/or by using micro-organisms that lack the ability to synthesise the necessary enzymes. However in order to obtain substantial yields of polymer a period of cultivation under conditions of limitation, and preferably depletion, of a nutrient required for growth is generally desirable.

The fermentation is preferably conducted so that the amount of accumulated polymer comprises about 50 to 80% by weight of the bacterial cells.

Acids that can be used to produce the copolymers should be those that do not give rise only to HB repeat units, when the cultivation is in the growth limited state. Unsuitable acids therefore include acetic and β-hydroxybutyric acids, members of the TCA cycle, and acids giving only acetyl CoA and/or a member of the TCA cycle when the cultivation is in the growth limited state. Thus unsuitable acids also include phosphoglyceric, pyruvic, citric, isocitric, α-ketoglutaric, succinic, fumaric, maleic, malic, oxalacetic, oxalosuccinic, aconitic, and methyl malonic, acids. Amino acids are likewise unsuitable. Butyric acid, which undergoes β-oxidation to β-hydroxybutyric acid, is likewise unsuitable. Formic acid does not give copolymers because the enzyme thiokinase does not add coenzyme A to formate.

As mentioned hereinbefore, in some cases the micro-organism may perform further reactions on the acid: thus isobutyric acid would be expected to give 3-hydroxy-4-methylpentanoate repeat units. In fact HV repeat units are found indicating that the micro-organism here substitutes hydrogen for a methyl group during the metabolic pathway to the copolymer.

Suitable acids include propionic, isobutyric, 3-chloropropionic, 3-hydroxypropionic, acrylic, methacrylic (i.e. 2-methyl acrylic), 3,3-dimethylacrylic and 2,3-dimethyl acrylic acids.

The substrate should be water soluble and so the acid may be added as such if water soluble or as a water soluble salt, e.g. an alkali metal, salt thereof.

Micro-organisms that may be used include any PHB accumulating micro-organisms that are capable of assimilating the acid (or salt thereof) to produce the copolymers. The bacteria *Alcaligenes eutrophus* (previously known as *Hydrogenomonas eutropha*) species, e.g. strain H 16 widely employed in academic studies of this species, see e.g. J General Microbiology (1979) 115 pages 185—192, and which is available as ATCC strain 17699, and mutants of strain H 16 such as mutants 11/7B, S301/C5, S501/C29 and S501/C41, which have been deposited, on 18 August 1980 with the National Collection of Industrial Bacteria, Torry Research Station, Aberdeen, Scotland, under NCIB Nos. 11600, 11599, 11597 and 11598 respectively, are particularly suitable. The ATCC number refers to the number designated by the American Type Culture Collection, 12301 Park Lawn Drive, Rockville, Maryland 20852, U.S.A. As mentioned hereinbefore a carbohydrate is preferably used as the substrate during the growth stage. While *Alcaligenes eutrophus* strain H 16 (ATCC 17699) will not utilise glucose, certain mutants thereof, e.g. the aforesaid mutants 11/7B, S301/C5, S501/C29 and S501/C41 can utilise glucose. Carbohydrates, particularly glucose, are the preferred substrates for the growth stage in view of the cost and the fact that the micro-organisms can growth efficiently thereon.

The polymer is produced as granules inside the micro-organism cells. While the cells containing the polymer may themselves be used as a moulding material, for example as described in USP 3,107,172, it is generally desirable to separate the polyester from the bacterial cells. This may be accomplished by subjecting the cells to a cell breakage step followed by extraction of the polyester with a suitable solvent. Examples of suitable extraction processes are described in our European Patent Application 15123.

As mentioned hereinbefore the copolymers should have a weight average molecular weight (Mw) above 10,000 as measured by gel permeation chromatography, if they are to be of any practical use. Preferably Mw is above 50,000, more preferably above 100,000 and in particular above 200,000.

The copolymers invariably have the D-configuration and exhibit melting points below that of PHB.

The copolymers are of particular utility in the preparation of melt-fabricated articles, where their reduced crystallinity compared to PHB is often advantageous.

Of particular interest is the use of small amounts of the copolymers as high molecular weight processing aids for vinyl chloride polymers. For this application the amount of copolymer is preferably 0.5 to 10% by weight of the vinyl chloride polymer. For the best results in this application, we have found that the copolymer should be random: to obtain random copolymers, the acid used to produce the HV units is preferably the sole substrate present, at least throughout the period of cultivation of the micro-organism under the conditions of growth requirement limitation.

Copolymers are also of particular utility in the production of film by melt extrusion, preferably followed by rolling, e.g. passage through one or more pairs of rolls, to reduce the film thickness and induce some orientation, at a temperature between the glass transition temperature (Tg) and the melting point of the polymer.

The invention is illustrated by the following examples.

Example 1 (comparative)

*Alcaligenes eutrophus* mutant NCIB 11599 was grown by aerobic cultivation at pH 6.8 and 34°C in a 5 litre batch fermenter containing 4000 ml of an aqueous medium A having the composition, per litre of deionised water:

| | |
|---|---|
| $(NH_4)_2SO_4$ | 4 g |
| $MgSO_4 . 7H_2O$ | 0.8 g |
| $K_2SO_4$ | 0.45 g |
| $H_3PO_4$ (1.1 M) | 12 ml |
| $FeSO_4 . 7H_2O$ | 15 mg |
| Trace element solution | 24 ml |

The trace element solution had the following composition, per litre of deionised water

| | |
|---|---|
| $CuSO_4 . 5H_2O$ | 0.02 g |
| $ZnSO_4 . 6H_2O$ | 0.1 g |
| $MnSO_4 . 4H_2O$ | 0.1 g |
| $CaCl_2 . 2H_2O$ | 2.6 g |

Glucose was fed to the fermenter at a rate of 8 g $hr^{-1}$. The amount of assimilable nitrogen in medium A was sufficient to support 26 g of PHB-free cells.

After 40 hours the cells were harvested by centrifugation; they were then freeze dried and the polymer extracted with chloroform.

Example 2

Example 1 was repeated except that when the cell weight reached 34 g, propionic acid was fed to the fermenter instead of glucose at a rate of 2.8 g $hr^{-1}$.

Example 3

Example 2 was repeated except that feed of propionic acid was commenced when the cell weight reached 39 g.

Example 4

Example 2 was repeated except that the feed of propionic acid was commenced when the cell weight reached 56 g.

Example 5

Example 2 was repeated except when the cell weight reached 48 g a single addition of 12 g of propionic acid was made.

Example 6

Example 1 was repeated except that the medium contained only 2 g $l^{-1}$ of ammonium sulphate and propionic acid was fed at a rate of 4 g $hr^{-1}$ instead of glucose throughout the fermentation.

Example 7

Example 1 was repeated except that when the cell weight reached 38 g, a mixture of glucose and propionic acid was fed to the fermenter, instead of glucose, at a rate of 5.2 g $hr^{-1}$ glucose and 2.8 $hr^{-1}$ propionic acid.

Example 8

Example 7 was repeated except that the mixture of glucose and propionic acid was fed at a rate of 6.8 g $hr^{-1}$ glucose and 1.2 g $hr^{-1}$ propionic acid, commencing when the cell weight reached 28 g.

In Examples 1 to 8 the propionic acid was added as a solution containing 400 g $l^{-1}$.

Example 9

Example 1 was repeated except that when the cell weight reached 28 g, isobutyric acid was fed to the fermentation vessel at a rate of 2 g $hr^{-1}$ in place of glucose. The isobutyric acid was added as a solution containing 150 g $l^{-1}$.

In Examples 2—5 and 7—9 the fermentations were continued until the ratio of the weight of acid fed to the fermenter to the sum of the weight of glucose fed to the fermenter after the cell weight had reached 26 g, i.e. when the system became nitrogen depleted, and the weight of acid fed to the fermenter, reached the values set out in Table 1.

Example 10

Example 1 was repeated except that when the cell weight reached 26.4 g, 3-chloropropionic acid was fed to the fermenter instead of glucose at a rate of 4 g $hr^{-1}$ for 5 hours.

Example 11

Example 10 was repeated except that feed of 3-chloropropionic acid was commenced when the cell weight reached 34.4 g.

Example 12

Example 11 was repeated except that a single addition of 4 g of 3-chloropropionic was made when the cell weight reached 30 g and then glucose was fed at a rate of 6.8 g $hr^{-1}$ for 7 hours.

In Examples 10—12 the 3-chloropropionic acid was added as a solution containing 50 g $l^{-1}$.

TABLE 1

| Example | Acid | Acid feed ratio* (%) | Final cell concentration (g . $l^{-1}$) | Amount of polymer in cells (% by weight) |
|---|---|---|---|---|
| 1 | none | 0 | 20.0 | 70 |
| 2 | propionic | 75 | 15.6 | 70 |
| 3 | propionic | 50 | 13.3 | 60 |
| 4 | propionic | 33 | 16.0 | 70 |
| 5 | propionic | 4 | 13.0 | 63 |
| 6 | propionic | 100 | 6.4 | 55 |
| 7 | propionic | 17 | 13.6 | 55 |
| 8 | propionic | 9.5 | 14.2 | 67 |
| 9 | isobutyric | 66 | 13.0 | 50 |
| 10 | 3-chloropropionic | 61 | 7.4 | 25 |
| 11 | 3-chloropropionic | 33 | 4.5 | 20 |
| 12 | 3-chloropropionic | 6.5 | 9.3 | 35 |

*acid feed ratio is defined as the weight of acid fed to the fermenter divided by the sum of the weight of glucose added after the cell dry weight reached 26 g and the weight of acid fed to the fermenter.

The amount of comonomer units in the polymers of Examples 1 to 12 was determined (a) by hydrolysis and gas chromatography and (b) by $^{13}C$ nuclear magnetic resonance spectroscopy.

The molecular weights of the polymers were determined by gel permeation chromatography.

Chlorine analyses were also performed on the polymers of Examples 1, 10, 11 and 12.

The results are shown in Table 2.

It is seen that little of the chlorine from 3-chloropropionic acid is to be found in the polymer. It would therefore appear that during the metabolism of 3-chloropropionic acid, HCl is lost and the resulting carbon-carbon double bond is hydrogenated. However the chlorine content of the polymers of Examples 10—12 may indicate that some of the chlorine is present as 2-chloroethyl groups.

TABLE 2

| Example | Acid employed | Mole % HV units | | Mole % units other than HB and HV units (by NMR) | Molecular weight | | chlorine ppm. |
|---|---|---|---|---|---|---|---|
| | | by NMR | by hydrolysis and gas chromatography | | $MW \times 10^{-3}$ | Mw/Mn | |
| 1 | none | 0 | 0 | | 292 | 2.75 | 40 |
| 2 | propionic | 27 | 33 | | 207 | 4.23 | |
| 3 | propionic | 24 | 27 | | 374 | 1.89 | |
| 4 | propionic | 13 | 14 | | 258 | 3.50 | |
| 5 | propionic | 6 | 3 | | 348 | 1.66 | |
| 6 | propionic | 25 | 26 | | 336 | 1.70 | |
| 7 | propionic | 15 | 14 | | 389 | 1.67 | |
| 8 | propionic | 6 | 7 | | 243 | 2.56 | |
| 9 | isobutyric | 30 | 29 | | 274 | 2.38 | |
| 10 | 3-chloropropionic | 7 | — | 1.8 | 383 | 2.99 | 475 |
| 11 | 3-chloropropionic | 4 | — | 1.2 | 376 | 1.77 | 265 |
| 12 | 3-chloropropionic | 2 | — | 0.6 | 311 | 1.99 | 45 |

High resolution $^{13}C$ NMR was used to investigate the monomer sequences of the copolymers of Examples 2—9. The signal derived from the carbon atom of the carbonyl group was found to occur at different chemical shifts depending upon its environment. Thus the possible sequences are

A. HB—HB
B. HV—HV
C. HB—HV

NMR examination of the polymers of Examples 2—9 revealed three resonances occurring at 169.07, 169.25 and 169.44 ppm respectively. Following the work of M Iida et al (Macromolecules *11*, 1978, p 490) the resonance at 169.07 ppm can be assigned to the HB—HB sequence A, and that at 169.44 ppm to the HV—HV sequence B. By inference the signal at 169.25 must arise from the HB—HV sequence C.

Quantitative analysis of the NMR results of the copolymer of Example 9 gave the following results:

| | |
|---|---|
| sequence A (HB—HB) | 55% |
| sequence B (HV—HV) | 14% |
| sequence C (HB—HV) | 31% |

These results clearly indicate that the polymer of Example 9 contains a substantial amount of a HB/HV copolymer. However it is possible that some HB homopolymer is also present.

All the polymers of Examples 1—12 had the D(−) configuration.

The melting behaviours of the copolymers as extracted was first determined by differential scanning calorimetry (DSC) using the DuPont 1090 system with computerised data analysis. DSC was also performed on the samples after compression moulding at 190°C and slow cooling in the press in order to obtain a fully crystallised product. In each case the specimens were heated at 20°C/min in air and the temperatures at the start (Ts) and peak (Tp) of the melting endotherm, together with its area, were noted. Heating of the annealed sample was continued to 200°C and, after isotherming for one minute to ensure complete melting, it was quenched in liquid nitrogen. The DSC was then re-run in order to determine the glass transition temperature (Tg) of the amorphous phase. Finally the densities of the annealed copolymers were measured by flotation in a density gradient.

The results are shown in Table 3.

TABLE 3

| Example | DSC on extracted polymer | | | DSC on annealed polymer | | | | Density $g . cm^{-3}$ |
|---|---|---|---|---|---|---|---|---|
| | Ts °C | Tp °C | Area $J . g^{-1}$ | Tg °C | Ts °C | Tp °C | Area $J . g^{-1}$ | |
| 1 | 140 | 183 | 100 | 5.9 | 140 | 191 | 127 | 1.256 |
| 2 | 120 | 125<br>166 | 5<br>20 | −1.9 | 140 | 171 | 18 | 1.172 |
| 3 | 120 | 170 | 50 | 0.8 | 140 | 182 | 44 | 1.174 |
| 4 | 110 | 120<br>170 | 5<br>50 | 2.2 | 140 | 177 | 45 | 1.200 |
| 5 | 120 | 172 | 100 | 2.7 | 120 | 173 | 96 | 1.225 |
| 6 | 80 | 132 | 34 | 0.4 | 80 | 132 | 40 | 1.198 |
| 7 | 110 | 120<br>166 | 6<br>60 | 2.0 | 140 | 174 | 43 | 1.199 |
| 8 | 110 | 156 | 89 | 4.0 | 110 | 163 | 81 | 1.210 |
| 9 | 50 | 65<br>120<br>168 | 10<br>3<br>25 | −2.0 | 130 | 172 | 26 | 1.138 |
| 10 | 110 | 170 | 57 | 5.0 | 120 | 180 | 73 | — |
| 11 | 110 | 177 | 86 | 4.1 | 120 | 173 | 86 | 1.182 |
| 12 | 100 | 172 | 98 | 5.9 | 120 | 171 | 96 | 1.218 |

The wide melting ranges of the copolymers indicated that the copolymers were of rather heterogeneous composition. However significant randomisation by ester interchange occurred on annealing since the melting endotherms became much sharper and slightly reduced in area. This is indicative that the polymers are not physical blends of homopolymers but are genuine copolymers.

Multiple DSC peaks were observed for the "as extracted" polymers of Examples 2, 4, 7 and 9.

The area of the melting endotherms gives an indication of the degree of crystallinity. All the polymers of Examples 2 to 12 after annealing were significantly less crystalline than the control homopolymer of Example 1.

Example 13

*Alcaligenes eutrophus* mutant NCIB 11599 was grown by aerobic cultivation at pH 6.8 and 34°C in a 5 litre batch fermenter containing 4000 ml of an aqueous medium B which was the same as medium A except that the amount of ammonium sulphate was 5.2 g $l^{-1}$ which is sufficient to support 8.5 g $l^{-1}$ of PHB-free cells.

The substrate was glucose supplied at a rate of 5.5 $l^{-1}$ $hr^{-1}$. When the cell concentration reached 7 g $l^{-1}$, propionic acid was fed at a rate of 1.58 g $l^{-1}$ $hr^{-1}$ in addition to the glucose. The cells were harvested when the cell dry weight reached 15 g $l^{-1}$. The cell suspension was spray dried, lipids extracted by refluxing the dried cells with methanol, and the polymer then extracted by refluxing with chloroform. The polymer was recovered by precipitation by adding the chloroform solution to a methanol/water mixture.

The copolymer contained 20 mole % of HV repeat units. The copolymer had a molecular weight of 350,000, and was insoluble in cold methyl ethyl ketone. When 2 g of the copolymer was refluxed with 100 ml of methyl ethyl ketone for 1 hour it all dissolved: on cooling the solution a gelatinous mass was formed. In contrast less than 0.1 g of a β-hydroxybutyric acid homopolymer dissolved when 2 g of the homopolymer was refluxed with 100 ml of methyl ethyl ketone. When these solubility tests were repeated with ethanol in place of methyl ethyl ketone, about 0.7 g of the copolymer, and less than 0.04 g of the homopolymer, had dissolved after refluxing for 1 hour.

The solubility of the copolymer in ethanol was also assessed at a lower concentration: thus 0.5 g of the copolymer was refluxed with 1 litre of ethanol for 1 hour. Less than 0.2 g of the copolymer dissolved.

In contrast it is noted that the polymers described by Wallen et al in "Environmental Scienic and Technology" *8* (1974) pages 576—579 were said to be soluble in hot ethanol.

Example 14

Aqueous media C, D, and E were made up to the following compositions, per litre of deionised water:

| | |
|---|---|
| **Medium C** | |
| $(NH_4)_2SO_4$ | 12 g |
| $MgSO_4 . 7H_2O$ | 1.2 g |
| $K_2SO_4$ | 1.5 g |
| $CaCl_2$ | 0.12 g |
| $FeSO_4 . 7H_2O$ | 0.1 g |
| $ZnSO_4 . 7H_2O$ | 0.006 g |
| $MnSO_4 . 4H_2O$ | 0.006 g |
| $CuSO_4 . 5H_2O$ | 0.0015 g |
| $H_2SO_4$ (concentrated) | 1 ml |
| **Medium D** | |
| $H_3PO_4$ (1.1 M) | 2.4 ml |
| glucose | 40 g |
| **Medium E** | |
| $H_3PO_4$ (1.1 M) | 2.4 ml |
| propionic acid | 40 g |

A sterilised batch fermenter of nominal capacity 250 litres was filled to the 130 litre mark with a mixture

of approximately equal volumes of media C and D. A small sample of the medium in the fermenter was then analysed for nitrogen content. The fermenter was then innoculated with *Alcaligenes eutrophus* mutant NCIB 11599 and fermentation conducted aerobically at 34°C with automatic pH control at 6.8 by addition of a sodium hydroxide solution.

The amount of assimilable nitrogen present in the fermenter was sufficient to support the growth of the micro-organism to only about 1.2 kg of polymer-free cells. When the cell weight reached about 1.05 kg feed of medium D was commenced at a rate of 6.5 litres/hour.

When the weight of cells reached approximately 1700 g feed of medium D was stopped and feed of medium E commenced at a rate of 6.5 litres/hour, and fermentation continued until about 2.6 kg of cells had been produced.

The cell suspension was then concentrated by centrifugation to a concentration of about 60 g/litre and the polymer extracted therefrom by contacting 1 volume of the suspension with 2 volumes of 1,2-dichloroethane (DCE) in a Silverson mixer at 20°C for 15 minutes. The DCE phase was separated from the aqueous phase, which contained the cell debris, and filtered. The polymer was precipitated by adding 1 volume of the filtered DCE phase to 4 volumes of a methanol/water mixture (4 volumes of methanol to 1 volume of water). The precipitated polymer was collected by filtration, washed with methanol, and dried in an oven for 4 hours at 100°C.

The polymer had a melting range, as determined by differential scanning calorimetry, of about 100°C to 180°C with a peak in the melting endotherm at 168°C.

Example 15

The fermentation procedure of Example 14 was repeated except that the changeover from feeding medium D to feeding medium E took place when the weight of cells was approximately 3.5 kg. The medium E was fed at a rate of 11.4 litres/hour for 4 hours and then reduced to 3.2 litres/hour and maintained at this level for a further 9 hours at which stage the weight of cells was about 3.9 kg.

In this example the amount of assimilable nitrogen present in the fermenter was sufficient to support the growth of the micro-organism to only about 1.5 kg of polymer-free cells.

The cell suspension was concentrated by centrifugation and then the polymer was extracted from the concentrated cell suspension by the procedure described in Example 13.

Example 16

A 250 litre fermenter was charged and innoculated as in Example 14. The amount of assimilable nitrogen was sufficient to support growth of the micro-organisms only to about 1.9 kg of polymer-free cells. As in Example 14 fermentation was conducted aerobically at 34°C at a pH of 6.8.

When the cell weight reached about 1.0 kg feeds of medium D and of a medium F at rates of 8.7 litres/hour and 4.6 litres/hour respectively were commenced and continued until the cell weight reached about 3.9 kg.

Medium F had the composition, per litre of deionised water:

| | |
|---|---|
| $H_3PO_4$ (1.1 M) | 1.2 ml |
| propionic acid | 20 g |

The cell suspension was concentrated by centrifugation and then the polymer extracted from the concentrated cell suspension by the procedure described in Example 13.

Example 17

The procedure of Example 15 was repeated on a larger scale using a fermenter of nominal capacity 1000 litres which was filled to the 500 litre mark with approximately equal volumes of medium C and D. In this example the feed of medium D was commenced, at a rate of 25 litres/hour, when the weight of cells was about 4 kg and the feed of medium E was commenced, at a rate of 37.5 litres/hour, when the weight of the cells was about 8 kg. The feeds of media were continued until the weight of cells was about 10 kg. The amount of assimilable nitrogen present was sufficient to support the growth of the micro-organism to only about 4.1 kg of polymer-free cells.

Example 18

Example 17 was repeated except that the feed rate of medium E was 25 litres/hour and the fermentation was continued until the weight of the cells was about 11 kg. In this case the amount of assimilable nitrogen present was sufficient to support the growth of the micro-organism to only about 4 kg of polymer-free cells.

The polymers of Examples 14—18 were each HB/HV copolymers and had weight average molecular weights above 300,000. They each had the D(−) configuration.

100 parts by weight of each of the polymers of Examples 14—18, and of a HB homopolymer, were slurried with about 10 parts by weight of chloroform and 1 part by weight of steamic talc, and granulated at room temperature through a domestic mincer. The compositions were then dried to remove the chloroform and extruded at 190°C and regranulated. The resulting granules were injection moulded at 185°C into test bars using a mould temperature of 60°C and a cooling time of 20 sec. The tensile properties were measured according to ASTM D 638-77a at a rate of 50 mm/min and the impact strength assessed by the Izod impact test according to ASTM D 256-78.

The results are shown in Table 4.

TABLE 4

| Example | HV/HB molar ratio | | Modulus* (GPa) | Tensile strength (MPa) | Extension to break (%) | Izod impact strength (J/m) | |
|---|---|---|---|---|---|---|---|
| | by GC | by NMR | | | | 1 mm notch | unnotched |
| 14 | 18/82 | 20/80 | 1.47 | 25 | 10—31 | 66 | 463 |
| 15 | 4/96 | 6/94 | 2.98 | 33 | 5—7 | 23 | 140 |
| 16 | 8/92 | 7/93 | 2.10 | 31 | 14—19 | 106 | 408 |
| 17 | 1/99 | 4/96 | 2.70 | 35 | 8—14 | 56 | 191 |
| 18 | 4/96 | 4/96 | 2.48 | 35 | 8—15 | 23 | 140 |
| homopolymer | 0/100 | 0/100 | 3.25 | 40 | 6—13 | 65 | 115 |

*at 0.5% extension

Example 19

A PVC formulation was made by dry blending the following ingredients at room temperature:

| | parts by weight |
|---|---|
| (i) vinyl chloride homopolymer (K62) | 100 |
| (ii) a complex tin thiooctyl stabiliser bases on a di-N-dithioglycollic acid ester | 1.5 |
| (iii) methyl methacrylate/butadiene/styrene PVC impact modifier | 8 |
| (iv) wax (external lubricant) | 0.8 |
| (v) glyceryl monoester (internal lubricant) | 1 |
| (vi) HB polymer (processing aid) | 2 |

The HB polymer processing aids were
a) a HB homopolymer prepared by the procedure of Example 1
b) the copolymer of Example 6 (copolymer A)
c) the copolymer of Example 14 (copolymer B)

The processing aids were slurried with about 10% by weight of chloroform, granulated at room temperature through a domestic mincer, dried, melt extruded at 190°C, regranulated, and ground to a particle size below 150 μm before incorporation into a PVC dry blend.

The dry blends were tested as follows:

1. 50 g of the mixture was poured into the mixing head of a Brabender Plastograph maintained at 180°C rotating at 18 rpm under a pressure ram loaded with a 5 kg weight. The time taken for gelation to occur was measured.

2. The mixture was cold compressed to form a candle which was then charged to an extrusion rheometer maintained at 170°C and fitted with a die having a circular orifice of 1 mm diameter and 20 mm land length. After the charge had heated to 170°C, it was extruded at increasing rates. The appearance of the extrudate was noted and the melt extensibility assessed by attempting to draw the extrudate away from the die.

The results are shown in Table 5.

TABLE 5

| Processing aid | Gelation time (min) at 180°C | Extrusion at 170°C | |
|---|---|---|---|
| | | Appearance | Melt extensibility |
| None | 12 | Severe sharkskin at low extrusion rates; ripple at higher rates | Poor |
| homopolymer | 9.5 | Poor with a lot of unmelted polymer clearly visible | Poor |
| copolymer A | 1.0 | Excellent — very smooth | Good |
| copolymer B | 1.5 | Smooth, but occasional unmelted particles | Fair |

This example shows that the copolymers are superior to the HB homopolymer as a vinyl chloride polymer processing aid. The more random copolymer A was clearly superior to the copolymer B.

Example 20

A medium G was made up to the following composition:

| | |
|---|---|
| $(NH_4)_2SO_4$ | 1 g |
| $KH_2PO_4$ | 2 g |
| $(Na)_2HPO_4$ | 3 g |
| $MgSO_4 . 7H_2O$ | 0.2 g |
| $CaCl_2$ | 0.01 g |
| $FeSO_4 . 7H_2O$ | 0.005 g |
| $MnSO_4 . 4H_2O$ | 0.002 g |
| $Na_2CO_3 . 10H_2O$ | 0.1 g |
| $(NH_2)_2CO$ | 1.5 g |
| deionised water | —to 1 litre |

This medium had a pH of 7.

Eight 1 litre shake flasks each containing 500 ml of medium G, in which 0.5 g of methacrylic acid had been dissolved, were each innoculated with 5 ml of a starter culture of *Nocardia salmonicolor* strain ATCC 19149 and incubated at 32°C on a gyratory shaker.

0.5 g of methacrylic acid was added to each flask at intervals of 24, 48 and 72 hours after innoculation, and a final addition of 0.25 g of methacrylic acid was made 96 hours after innoculation. After a total of 108 hours after innoculation the flasks were examined: little or no growth of the microorganism was apparent in any of the flasks. The contents of the flasks were combined and centrifuged to give a pellet of cells which was dried in an oven and weighed. The weight of the pellet was 2.81 g. The cell content of the innoculum was also determined and found to be 69.75 g . $l^{-1}$: Hence the total weight of cells added, as the innoculum, to the flasks was 2.79 g.

It is concluded that, at the concentrations of methacrylic acid employed, this strain does not assimilate methacrylic acid.

**Claims**

1. β-hydroxybutyrate copolymers having a weight average molecular weight above 50,000 and containing 50—99 mole % of β-hydroxybutyrate repeat units and 1—50 mole % of β-hydroxyvalerate repeat units.

2. β-hydroxybutyrate copolymers having the D(–) configuration, a weight average molecular weight above 10,000, and containing 50—99 mole % of β-hydroxybutyrate repeat units and 1—50 mole % of β-hydroxyvalerate repeat units.

3. β-hydroxybutyrate copolymers according to claim 1 or claim 2 having a weight average molecular weight above 200,000.

4. β-hydroxybutyrate copolymers according to any one of claims 1 to 3 containing 1 to 40 mole % of β-hydroxyvalerate repeat units.

5. β-hydroxybutyrate copolymers according to claim 4 containing 3 to 30 mole % of β-hydroxyvalerate repeat units.

**Patentansprüche**

1. β-Hydroxybutyrat-Copolymere, die ein Durchschnittsmolekulargewicht (Gewichtsmittel) von mehr als 50.000 haben und 50 bis 99 Mol-% β-Hydroxybutyrat-Struktureinheiten und 1 bis 50 Mol-% β-Hydroxyvalerat-Struktureinheiten enthalten.

2. β-Hydroxybutyrat-Copolymere, die die D(–)-Konfiguration und ein Durchschnittsmolekulargewicht (Gewichtsmittel) von mehr als 10.000 haben und 50 bis 99 Mol-% β-Hydroxybutyrat-Struktureinheiten und 1 bis 50 Mol-% β-Hydroxyvalerat-Struktureinheiten enthalten.

3. β-Hydroxybutyrat-Copolymere nach Anspruch 1 oder 2, die ein Durchschnittsmolekulargewicht (Gewichtsmittel) von mehr als 200.000 haben.

4. β-Hydroxybutyrat-Copolymere nach einem der Ansprüche 1 bis 3, die 1 bis 40 Mol-% β-Hydroxyvalerat-Struktureinheiten enthalten.

5. β-Hydroxybutyrat-Copolymere nach Ansprüche 4, die 3 bis 30 Mol-% β-Hydroxyvalerat-Struktureinheiten enthalten.

**Revendications**

1. Copolymères β-hydroxybutyriques ayant un poids moléculaire moyen en poids supérieure à 50000 et contenant 50 à 99% en moles d'unités répétées β-hydroxybutyrate et 1 à 50% en moles d'unités répétées β-hydroxyvalérate.

2. Copolymères β-hydroxybutyriques ayant la configuration D(−), un poids moléculaire moyen en poids supérieur à 10000 et contenant 50 à 99% en moles d'unités répétées β-hydroxybutyrate et 1 à 50% en moles d'unités répétées β-hydroxyvalérate.

3. Copolymères β-hydroxybutyriques suivant la revendication 1 ou la revendication 2, ayant un poids moléculaire moyen en poids supérieur à 200000.

4. Copolymères β-hydroxybutyriques suivant l'une quelconque des revendications 1 à 3, contenant 1 à 40% en moles d'unités répétées β-hydroxyvalérate.

5. Copolymères β-hydroxybutyriques suivant la revendication 4, contenant 3 à 30% en moles d'unités répétées β-hydroxyvalérate.